# EUROPÄISCHE PATENTSCHRIFT

(11) **EP 2 470 630 B1**
(45) Veröffentlichungstag und Bekanntmachung des Hinweises auf die Patenterteilung: **11.10.2017**
(21) Anmeldenummer: 10747216.9
(22) Anmeldetag: 27.08.2010
(51) Int. Cl.: C11D 1/65, C11D 1/83, C11D 1/94, C11D 3/00, A61K 8/39, A61K 8/42, A61K 8/45, A61Q 5/02, A61Q 19/10, C11D 1/10, C11D 1/14, C11D 1/29, C11D 1/52, C11D 1/74, C11D 1/825, C11D 1/835

(54) **PERLGLANZKONZENTRAT UND VERFAHREN ZUR HERSTELLUNG**
PEARLESCENT CONCENTRATE AND METHOD FOR PRODUCING THE SAME
CONCENTRÉ NACRÉ ET PROCÉDÉ DE PRODUCTION

(30) Priorität: 27.08.2009 DE 102009040455
(43) Veröffentlichungstag der Anmeldung: 04.07.2012
(73) Patentinhaber: Clariant International Ltd, 4132 Muttenz (CH)
(72) Erfinder: DAHMS, Gerd, H., 47138 Duisburg (DE); JUNG, Andreas, H., 47198 Duisburg (DE)
(74) Vertreter: Holmes, Rosalind
(86) Internationale Anmeldenummer: PCT/EP2010/062576
(87) Internationale Veröffentlichungsnummer: WO 2011/023803

(56) Entgegenhaltungen:
- WO-A1-99/09944
- DE-A1- 3 617 306
- DE-C1- 19 705 862
- US-A1- 2005 158 270

## Beschreibung

Die Erfindung betrifft Verfahren zur Herstellung pumpbarer wässriger Perlglanzkonzentrate mit hohem Aktivgehalt an Alkylethersulfaten sowie Fettsäureglycolestern als Perlglanzbildner. Zur Verbesserung der optischen Erscheinung von Tensidkompositionen, zum Beispiel flüssigen Wasch- und Reinigungsmitteln (d. h. Bodenreinigungsmittel, Geschirrspülmittel oder ähnliche Produkte) oder flüssigen Körperreinigungs- und Körperpflegemitteln sowie Haarbehandlungsmitteln (d. h. Shampoos, Badezusätze, Flüssige Seifen, Duschgele oder ähnliche Produkte), werden diesen häufig wässrige Perlglanzdispersionen (Perlglanzkonzentrate) zugesetzt, wodurch den Tensidkompositionen ein Perlglanz und damit ein ästhetisch ansprechendes Äußeres verliehen wird.

Die bekannten wässrigen Perlglanzdispersionen bestehen im Wesentlichen aus mindestens einer perlglanzbildenden Verbindung (auch perlglanzbildende Komponente oder Perlglanzbildner genannt), mindestens einem Dispergiermittel (auch Lösemittel, Netzmittel oder Emulgator genannt) und Wasser. Im Stand der Technik werden zahlreiche perlglanzbildende Verbindungen und Verbindungsmischungen empfohlen, zum Beispiel Fettsäuren, Fettsäuremono- oder Fettsäuredialkanolamide, Monoester oder Diester von Alkylenglycolen wie Ethylenglycol, Propylenglycol oder Oligomeren davon mit Fettsäuren (Fettsäureglycolester) sowie Monoester oder Polyester von Glycerin mit höheren Fettsäuren. Noch zahlreicher sind die Verbindungen, die als Dispergiermittel beschrieben sind, zum Beispiel Alkylsulfate, Alkylethersulfate, Alkylsulfonate, Aminoxide, Betaine, Anlagerungsprodukte von Ethylenoxid und/oder Propylenoxid an Fettalkohole, Fettsäuren oder Alkylphenole sowie Glycerinmono- oder Glycerindiester bzw. Sorbitanmono- oder Sorbitandiester von Fettsäuren, wobei die Glycerinester und Sorbitanester gegebenenfalls ethoxyliert und/oder propoxyliert sind, Alkylmono- oder Alkyloligoglycoside und dergleichen.

Der augenblickliche Stand der Technik wird recht gut in den folgenden Patenten und Patentanmeldungen wiedergegeben.

Das US-Patent US 7,268,107 offenbart eine Perlglanzzusammensetzung, die von ca. 30 bis 55 Gew.-% eines perlglanzbildenden Wachses, ein nichtionisches Dispergiermittel, ggf. ein zwitterionisches Dispergiermittel, ca. 0,1 Gew.-% bis 5 Gew.-% eines Polyolesters und Wasser enthält, wobei der Aktivgehalt (Summe des Gehalts an perlglanzbildender Komponente und Dispergiermittel) mindestens ca. 55 Gew.-% bezogen auf das Gewicht der Zusammensetzung und die Summe des Gehalts an Dispergiermitteln weniger als 25 Gew.-% bezogen auf das Gewicht der Zusammensetzung betragen und wobei das nichtionische Dispergiermittel und der Polyolester in einem Gewichtsverhältnis von ca. 5:1 bis 10:1 vorhanden sind.

Die US-Patentanmeldung US 2004/0086470 A1 betrifft eine wässrige Perlglanzzusammensetzung, die etwa 30 bis 60 Gew.-% einer Wachskomponente, die zu mindestens 15 Gew.-% bezogen auf das Gewicht der Wachskomponente aus amorphen Partikeln und zu höchstens 85 Gew.-% bezogen auf das Gewicht der Wachskomponente aus kristallinen Partikeln besteht, und etwa 5 bis 25 Gew.-% eines Tensids, ausgewählt aus einem nichtionischen Tensid, einem amphoteren Tensid und Mischungen davon, enthält.

Das US-Patent US 6,727,217 B1 bezieht sich auf ein Verfahren zur Herstellung von perlglänzenden Tensidzusammensetzungen bei einer Temperatur von etwa 10 °C bis 45 °C, wobei eine wässrige Tensidlösung mit einer Zusammensetzung aus einem perlglanzbildenden Wachs und einem Polyolester kontaktiert wird.

Das US-Patent US 6,835,700 B1 offenbart ein fließfähiges Perlglanzkonzentrat, das etwa 25 bis 45 Gew.-% eines perlglanzbildenden Wachses, etwa 35 bis 40 Gew.-% eines nichtionischen, amphoteren, zwitterionischen und/oder kationischen Dispergiermittels, etwa 0,5 bis 15 Gew.-% eines Polyolesters und Wasser umfasst, wobei die Summe des Gehalts an Wachs, Dispergiermittel und Polyolester mindestens 55 Gew.-% beträgt.

Das US-Patent US 6,147,124 A betrifft fließfähige, biologisch abbaubare Perlglanzkonzentrate, die 5 bis 50 Gew.-% eines Alkanolethers oder eines Alkanolamids als perlglanzbildende Komponente, 5 bis 55 Gew.-% eines Fettsäure-M-alkyl-polyhydroxy-Alkylamids als alleiniges nichtionisches Dispergiermittel und 0,1 bis 40 Gew.-% eines niedermolekularen Polyols enthalten.

Das US-Patent US 6,235,702 B1 bezieht sich auf ein wässriges Perlglanzkonzentrat, das 1 bis 99 Gew.-% (bezogen auf das Gesamtgewicht) eines Esters einer Hydroxygruppentragenden Carbonsäure mit einem Fettalkohol, 0,1 bis 90 Gew.-% (bezogen auf das Gesamtgewicht) eines Dispergiermittels, ausgewählt aus einem anionischen Tensid, einem nichtionischen Tensid, einem kationischen Tensid, einem zwitterionischen Tensid, einem Esterquat und Mischungen aus diesen, und bis zu 40 Gew.-% (bezogen auf das Gesamtgewicht) eines Polyols enthält.

US 6,228,831 B1 offenbart ein Perlglanzkonzentrat, das 1 bis 99,9 Gew.-% einer Fettkomponente, ausgewählt aus der Gruppe bestehend aus Fettalkoholen, Fettketonen, Fettethern, Fettcarbonaten und Mischungen aus diesen, 1 bis 99,9 Gew.-% eines Dispergiermittels ausgewählt aus der Gruppe bestehend aus anionischen, nichtionischen, kationischen, ampholytischen und zwitterionischen Tensiden und Mischungen aus diesen, und bis zu 40 Gew.-% eines Polyols enthält.

US 5,711,899 betrifft fließfähige Perlglanzkonzentrate, die 15 bis 40 Gew.-% einer perlglanzbildenden Komponente, 5 bis 55 Gew.-% eines nichtionischen, ampholytischen und/oder zwitterionischen Tensids und 0,1 bis 5 Gew.-% eines niedermolekularen Polyols enthalten.

US 6,165,955 bezieht sich auf ein Perlglanzkonzentrat mit hoher Temperaturstabilität, das aus einem auf einer Fettsäure basierenden Perlglanzbildner ausgewählt aus der Gruppe bestehend aus Hydroxylstearaten, Polyethylenglycol-Mono- und Distearaten, Ethylenglycol-Mono- und Distearaten, Stearinsäure-Monoethanolamid und Mischungen aus diesen, besteht, wobei mindestens 90 Gew.-% der Fettsäuren des besagten Fettsäurebestandteils aus Octadecansäure bestehen.

Die europäische Patentanmeldung EP 0 568 848 A1 betrifft fließfähige wässrige Perlglanzdispersionen, die im wesentlichen aus einem Fettsäureglycolester als perlglanzgebende Komponente, aus zwei bestimmten tensidischen Verbindungen, nämlich einem Betain und einem Fettalkoholalkoxylat, und Wasser in einer jeweils bestimmten Menge bestehen.

Derartige Perlglanzformulierungen enthalten neben den in Tensidkompositionen als Schlüsselsubstanzen eingesetzten Alkylethersulfaten und/oder Alkylsulfaten und/oder Alkylsulfonaten noch eine Reihe von Hilfsstoffen wie z. B. Aminoxide, Betaine, Anlagerungsprodukte von Ethylenoxid und/oder Propylenoxid an Fettalkohole, Fettsäuren oder Alkylphenole, Glycerinmono- oder Glycerindiester oder Sorbitanmono- oder Sorbitandiester von Fettsäuren, wobei die Glycerinester und Sorbitanester gegebenenfalls ethoxyliert und/oder propoxyliert sind, Alkylmono- oder Alkyloligoglycoside, Alkohole, Polyole und dergleichen.

Die Konzentration der eingesetzten Schlüsselsubstanzen ist dabei in der Regel kleiner als 35 Gew.-%. Solche niedrigkonzentrierten Perlglanzformulierungen haben vorwiegend ökonomische Nachteile. Im direkten Vergleich zu den hochkonzentrierten Perlglanzformulierungen fallen hier insbesondere Transport-, Verpackungs- und Lagerkosten ins Gewicht. Die Herstellung von Perlglanzdispersionen erfolgt im Stand der Technik in der Regel diskontinuierlich in Rührreaktoren. Dabei werden die erforderlichen Mengen der Einsatzstoffe in ein Mischgefäß dosiert, auf 5 - 20°C oberhalb der Schmelztemperatur der am höchsten schmelzenden Komponente erhitzt und vermischt. Nach der Vermischung wird zunächst ohne zu Kühlen unter Rühren auf 30 - 40°C gekühlt. Erst nach Erreichen dieser Temperatur wird unter Anwendung von Kühlung auf Raumtemperatur abgekühlt.

Eine Kontrolle erfolgt in der Regel erst am fertigen Produkt der entsprechenden Mischungscharge. Eine kontinuierliche Überprüfung des Herstellungsprozesses ist in der Regel nicht möglich.

Desweiteren können durch die herkömmlichen Verfahren in der Regel keine hochkonzentrierten Perlglanzdispersionen hergestellt werden, da bedingt durch die hohe Viskosität derartiger Zusammensetzungen Probleme bei der Durchmischung der Komponenten auftreten. Aufgrund des für die Durchmischung erforderlichen hohen Energieeintrags durch das Rührwerk sind solche Verfahren einerseits sehrenergie- und kostenintensiv und andererseits wird das Rührgut sehr stark erwärmt, was wiederum die Qualität des Produkts negativ beeinflusst.

Darüber hinaus ist eine Variation der Produktmengen im Stand der Technik nur in sehr begrenztem Umfang möglich, da die mögliche Ansatzgröße bei einem Chargenmischer in einem eng begrenzten Bereich liegt. Die minimale Ansatzgröße darf in der Regel die Hälfte der maximalen Ansatzgröße nicht unterschreiten.

Die Patentanmeldung WO 2004/082817 A1 offenbart eine Vorrichtung und ein Verfahren zur kontinuierlichen Herstellung von Emulsionen oder Dispersionen. Die beschriebene Vorrichtung umfasst ein allseits geschlossenes Mischgefäß, das Zu- und Abführrohre zum Ein- und Austrag von fließfähigen Stoffen oder Stoffgemischen sowie ein Rührwerkzeug aufweist und das einen Rühreintrag in die Emulsion oder Dispersion ohne Erzeugung von Kavitationskräften und ohne Hochdruckhomogenisation erlaubt.

US 2005/0158270 A1 offenbart ein wässriges Perlglanzkonzentrat, aufweisend eine Perlglanzmittel, eine anionisches Tensid und eine Kationische Komponente, welches eine erhöhte Opazität aufweist und welches zur Veränderung der Erscheinungsbildes von wässrigen Körperpflegeproduktzusammensetzungen geeignet ist.

DE 36 17 306 A1 beschreibt ein Verfahren zur Herstellung einer hochkonzentrierten Perliermitteldispersion, die unter Erhöhung ihres Handelswerts beispielsweise in Shampoos, Spülmitteln, Kosmetika und Wasch-und Reinigungsmitteln Verwendung finden kann.

Das Verfahren ist dadurch gekennzeichnet, dass man 1. eine durch Erwärmen homogen gemachte hochkonzentrierte wässrige Lösung mit 6 - 45 Gew.-% eines Fettsäureglykolesters und 15 - 60% eines Netzmittels zu einer niedrigkonzentrierten Dispersion mit - in dispergiertem Zustand befindlichen -0,1 - 5 Gew.-% eines Fettsäureglykolesters zugibt und 2. das Gemisch zum Wachsen bzw. zur Vergrößerung des in der niedrigkonzentrierten Dispersion enthaltenen Perliermittels altert.

WO 99/09944 A1 offenbart wässrige Perlglanzkonzentrate, enthaltend bezogen auf den nicht-wässrigen Anteil, (a) 1 bis 99 Gew.-% Fettsäurepolyglycolestersulfate, (b) 0 bis 90 Gew.-% anionische, nichtionische, kationische, ampholytische und/oder zwitterionische Emulgatoren, (c) 1 bis 50 Gew.-% Perlglanzwachse sowie (d) 0 bis 40 Gew.-% Polyole, mit der Maßgabe, dass sich die Mengenangaben zu 100 Gew.-% ergänzen.

DE 197 05 862 C1 offenbart ein Verfahren zur Herstellung tensidischer Perlglanzkanzentrate mit einem Feststoffgehalt im Bereich von 60 bis 90 Gew.-% durch Vermischung wässriger anionischer Tenside mit Perlglanzwachsen vorgeschlagen, bei dem man wässrige Aniontensidkonzentrate einsetzt, die in der lamellaren Gelphase vorliegen. Durch Vermischen der Komponenten in der Gelphase werden Konzentrate erhalten, die sich durch einen brillanten Perlglanz auszeichnen.

Die Aufgabe der Erfindung besteht darin, ein neues Verfahren zur Herstellung für ein gut pumpbares, wässriges Perlglanzkonzentrat mit hohem Anteil an Aktivstoffen und ausgezeichnetem Perlglanzcharakter, das mit konventioneller Mischtechnologie, die auch für die Verarbeitung hochkonzentrierter konventionell eingesetzter Alkylethersulfate in Wasch- und Reinigungsmitteln geeignet ist, kalt verarbeitet und damit leicht gehandhabt werden kann, zur Verfügung zu stellen.

Die Aufgabe wird durch das Verfahren gemäß Anspruch 1 gelöst. Die erfindungsgemäß eingesetzten Dispergiermittel bzw. Perlglanzbildner können ein oder mehrere unterschiedliche Verbindungen aus den jeweiligen Gruppen enthalten.

Die Summe aus dem Gehalt an der perlglanzgebenden Verbindung und dem Gehalt an Dispergiermittel wird als Aktivgehalt des Perlglanzkonzentrats definiert und liegt bevorzugt bei >60 Gew.-%, bevorzugt bei 65 bis 85 Gew.-%, besonders bevorzugt bei 70 bis 80 Gew.-%.

Die Summe aus dem Gehalt des Perlglanzkonzentrats an Wasser und an Zusätzen ist vorzugsweise kleiner als 30 Gew.-%, bevorzugt 25 Gew.-%, bezogen auf die Gesamtmenge des Perlglanzkonzentrats.

Das erfindungsgemäß hergestellte wässrige Perlglanzkonzentrat hat vorzugsweise einen Gehalt von 35 bis 85 Gew.-%, bevorzugt 40 bis 70 Gew.-%, besonders bevorzugt 50 bis 60 Gew.-% eines oder mehrerer Alkylethersulfate, Alkylsulfate, Alkylsulfonate, Mono- und Diester der Sulfobernsteinsäure (Sulfosuccinate), Alkylglycinate, Alkylsarkosinate und/oder Alkyltaurate. Das Alkylethersulfat, Alkylsulfat, Alkylsulfonat, Sulfosuccinat, Alkylglycinat, Alkylsarkosinat und/oder Alkyltaurat wirkt dabei als Dispergiermittel, welches die erfindungsgemäße Perlglanzdispersion stabilisiert. Kommerziell erhältliche Alkylethersulfate, Alkylsulfate, Alkylsulfonate, Sulfosuccinate, Alkylglycinate, Alkylsarkosinate und/oder Alkyltaurate werden üblicherweise als wässrige Lösungen vertrieben und verarbeitet, wobei die Konzentrationen üblicherweise bei 70% wässrigen Lösungen oder darunter liegen.

Die Gesamtkonzentration an perlglanzbildender Komponente beträgt 15 bis 40 Gew.-% bezogen auf das Gesamtgewicht des Konzentrats. Bevorzugt beträgt die Gesamtkonzentration an perlglanzbildender Komponente in dem erfindungsgemäß hergestellten Perlglanzkonzentrat 15 bis 30 Gew.-%, besonders bevorzugt 20 bis 25 Gew.-%, bezogen auf das Gesamtgewicht des Konzentrats.

Das erfindungsgemäß hergestellte wässrige Perlglanzkonzentrat kann vorteilhafterweise ohne Schwierigkeiten in die üblichen Tensidkompositionen inkorporiert werden. Besonders bevorzugt erfolgt die Einarbeitung in die Tensidkomposition kalt, d. h. bevorzugt bei einer Temperatur unter 25°C, besonders bevorzugt bei Raumtemperatur. Bestandteil der Erfindung ist daher auch eine Tensidkomposition enthaltend ein erfindungsgemäß hergestelltes Perlglanzkonzentrat. Vorteilhafterweise verleiht auch schon der Einsatz einer relativ geringen Menge an Perlglanzkonzentrat der Tensidkomposition ein perlglänzendes Aussehen. Geeignete Anteile Perlglanzkonzentrat in einer Tensidkomposition liegen je nach gewünschtem Perlglanzeffekt zwischen 0,5 und 10 Gew.-%, bevorzugt zwischen 0,5 und 5 Gew.-%, besonders bevorzugt zwischen 0,5 und 2 Gew.-%.

Perlglanzdispersionen mit der erfindungsgemäßen hohen Konzentration an Aktivgehalt bieten sowohl ökologische als auch ökonomische Vorteile gegenüber konventionell verfügbaren Dispersionen. Der geringe Wassergehalt senkt sowohl die Transport- als auch die Lagerkosten beträchtlich. Besonders vorteilhaft ist, dass die erfindungsgemäßen Perlglanzkonzentrate konservierungsmittelfrei für die weitere Verarbeitung bereitgestellt werden können. Die Beschränkung des Dispergiermittels auf die ohnehin in Wasch- und Reinigungsmitteln eingesetzten Alkylsulfate, Alkylethersulfate, Alkylsulfonate, Mono- und Diester der Sulfobernsteinsäure (Sulfosuccinate), Alkylglycinate, Alkylsarkosinate und/oder Alkyltaurate lässt zudem dem Formulierer von Wasch- und Reinigungsmitteln vorteilhaft viel mehr Freiheiten in der endgültigen Zusammensetzung seines Produktes.

Die Erfindung basiert auf der überraschenden Feststellung, dass wässrige Alkylethersulfate, Alkylsulfate, Alkylsulfonate, Mono- und Diester der Sulfobernsteinsäure (Sulfosuccinate), Alkylglycinate, Alkylsarkosinate und/oder Alkyltaurate mit einem Gehalt von 40 bis 90 Gew.-% bezogen auf das Gesamtgewicht des Konzentrats sich mit perlglanzgebenden Verbindungen wie Fettsäureglycolestern und/oder Carbonsäureamiden zu pumpbaren Perlglanzkonzentraten verarbeiten lassen, deren Aktivgehalt oberhalb von 60 Gew.-%, bevorzugt bei 65 bis 85 Gew.-%, besonders bevorzugt bei 70 bis 80 Gew.-% liegt.

Als Gegenionen für die als Dispergiermittel verwendeten wässrigen Alkylethersulfate sind jegliche organische und anorganische Gegenkationen einsetzbar, so lange die Alkylethersulfate in Wasser leicht löslich oder dispergierbar bleiben und so lange die Gegenionen physikalisch und chemisch kompatibel mit den Grundbestandteilen des Perlglanzkonzentrats sind und die Produkteigenschaften wie Perlglanz, Stabilität oder Verarbeitbarkeit nicht nachteilig beeinflussen. Bevorzugt werden als Gegenionen Alkalimetallionen wie Natrium und/oder Kalium, Erdalkalimetallionen wie Calcium und/oder Magnesium sowie substituierte und nicht substituierte Ammoniumionen wie Monoethanolamin, Diethanolamin, Triethanolamin, Triisopropanolamin.

Als Alkylethersulfate werden bevorzugt die Sulfate von Fettalkoholen mit einer Kettenlänge von 8 bis 16 C-Atomen, z. B. Natriumlaurylethersulfat, Ammoniumlaurylethersulfat, Triethylaminlaurylethersulfat, Triethanolaminlaurylethersulfat, Magnesiumlaurylethersulfat, Natrium C12-13 Pareth Sulfat (Sodium C12-13 Pareth Sulfate), sowie das Na-Salz von Laurylmyristylethersulfat eingesetzt. Als Dispergiermittel werden Alkylethersulfate eingesetzt. Die im erfindungsgemäßen Perlglanzkonzentrat als perlglanzbildende Komponente enthaltenen Fettsäureglycolester entsprechen der allgemeinen Formel (I), wobei R₁ ein Alkylrest mit 12 bis 22 C-Atomen, R₂ ein zweiwertiger Rest der Formel -C₂H₄- oder -C₃H₅-, R₃ = H oder ein Rest der Formel 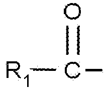 und n eine Zahl von 1 bis 10 ist.

R₁ ist bevorzugt ein gesättigter oder ungesättigter Alkylrest mit 12 bis 18 C-Atomen, R₂ ist bevorzugt -C₂H₄₋, R₃ ist bevorzugt R₁-CO- und n ist bevorzugt eine Zahl von 1 bis 3. Ist der Alkylrest R₁ ungesättigt, so enthält er vorzugsweise 1 bis 3 Doppelbindungen.

Besonders bevorzugte perlglanzbildende Komponenten sind Mono- und Diester des Ethylenglycols, Diethylenglycols oder Triethylenglycols mit Palmitinsäure, Stearinsäure, Ölsäure, Talgfettsäure, Cocosfettsäure und/oder anderen Fettsäuren, z. B. Ethylenglycolmonostearat, Ethylenglycoldistearat, Diethylenglycoldistearat.

Die im erfindungsgemäßen Perlglanzkonzentrat als perlglanzbildende Komponente enthaltenen Fettsäurealkanolamide entsprechen der allgemeinen Formel (II) wobei R₅ ein Alkylrest mit 8 bis 22 C-Atomen, R₆ = H, ein Alkylrest mit 1 bis 4 C-Atomen, 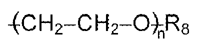 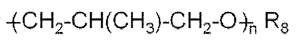 mit n = 1 bis 10, R₇ = H, ein Alkylrest mit 1 bis 4 C-Atomen, 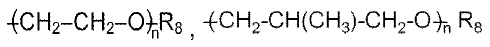 mit n = 1 bis10 und R₈ = H, Methyl, Ethyl, n-Propyl, i-Propyl, n-Butyl, i-Butyl oder t-Butyl ist.

Der perlmuttartige Glanz von Perlglanzdispersionen beruht auf dem Vorhandensein lamellarer lyotroper liquidkristalliner (auch als flüssigkristallin bezeichnet) Strukturen. Damit sich solche lamellaren Strukturen ausbilden können, muss ein optimales Verhältnis zwischen Emulgator und Perlglanzbildner bestehen, welches von der Geometrie der teilnehmenden Perlglanzbildner und Dispergiermittel abhängt. Der Parameter dafür ist der sogenannte kritische Packungsparameter (critical packing parameter). Wenn der kritische Packungsparameter Werte von ∼ 1 annimmt, hat man eine optimale Zusammensetzung gefunden und es herrschen optimale Bedingungen für die Bildung einer lamellaren Phase. Dem Fachmann sind Wege bekannt, den kritischen Packungsparameter für eine bestimmte Kombination von Perlglanzbildner und Dispergiermittel durch die Variation der eingesetzten Mengen einzustellen. Desweiteren sind dem Fachmann Mittel bekannt, mit welchen der kritische Packungsparameter gezielt geändert werden kann, wie z. B. die Zugabe eines langkettigen Alkohols, Amins oder eines anderen amphiphilen Moleküls oder die Zugabe eines Salzes. Bevorzugt enthalten die Perlglanzkonzentrate keine weiteren Tenside als die oben genannten Dispergiermittel und Perlglanzbildner

Die erfindungsgemäß hergestellten Perlglanzkonzentrate benötigen zur Ausbildung der lamellaren lyotrop liquidkristallinen Strukturen eine gewisse Menge Wasser, welches zwischen den lamellaren Doppelschichten aus den enthaltenen amphiphilen Molekülen (Dispergiermittel und Perlglanzbildner) eingelagert wird. Bevorzugt ist der Mindestgehalt an Wasser 5 Gew.-%, besonders bevorzugt 10 Gew.-%, ganz besonders bevorzugt 15 Gew.-%, bezogen auf das Gesamtgewicht des Konzentrats. Das lamellare System kann dabei abhängig von den verwendeten amphiphilen Substanzen eine bestimmte Menge Wasser aufnehmen, ein Prozess der als Quellung bezeichnet wird. Ist der Punkt erreicht, an dem das Konzentrat völlig durchgequollen ist und die Quellfähigkeit der im Konzentrat enthaltenen oberflächenaktiven Stoffe überstiegen wird, sammelt sich das freie Wasser, das nicht mehr gebunden werden kann, als sogenanntes "Bulkwasser".

Das erfindungsgemäß hergestellte Perlglanzkonzentrat verfügt vorzugsweise über ein hohes Phasenvolumenverhältnis, d. h. die Menge Wasser im Verhältnis zu den enthaltenen amphiphilen Molekülen ist gering. Dies gewährleistet zum einen die Vorteile, die mit einem hohen Aktivgehalt einhergehen (siehe oben), zum anderen ist die Ausbildung von lyotropen liquidkristallinen Lamellarstrukturen begünstigt. Dabei sind die Perlglanzkonzentrate vorzugsweise vollständig durchgequollen. Um dies sicherzustellen, ist der Wasseranteil bevorzugt so gewählt, dass er leicht über dem Maximum liegt, welches in den lamellaren Strukturen gebunden werden kann. Vorzugsweise weisen die erfindungsgemäßen Perlglanzkonzentrate einen Bulkwasseranteil von 0,5 bis 15 Gew.-%, bevorzugt 1 bis 10 Gew.-%, bezogen auf die Gesamtmenge des Perlglanzkonzentrats, auf.

Das Phasenvolumenverhältnisses kann mittels rheologischer Testmethoden bestimmt werden. Es ist weithin bekannt, dass die Viskosität mit steigender interner Phase exponentiell zunimmt. Das Viskositätsmaximum wird an dem Punkt erreicht, wenn keine Bulkwasserphase mehr existiert und alles Wasser interlamellar gebunden ist. Eine andere Testmethode für die Bestimmung des Phasenvolumenverhältnisses ist die Messung der Leitfähigkeit. Diese nimmt mit steigender interner Phase exponentiell ab und erreicht den Nullwert, wenn alles Wasser interlamellar gebunden ist.

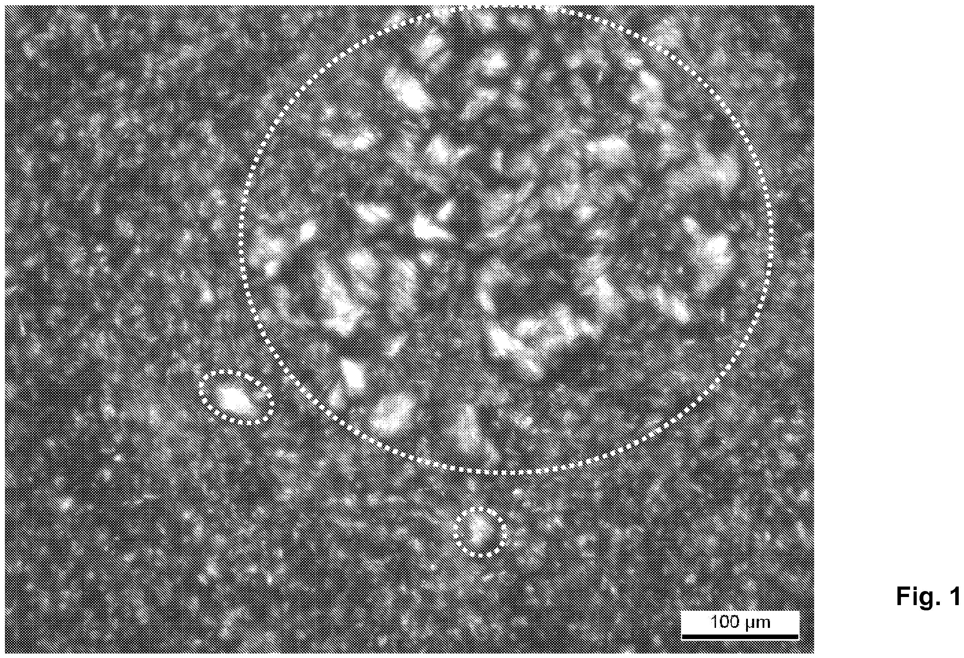

Die aus dem Stand der Technik bekannten Perlglanzdispersionen bestehen nicht vollständig aus gequollenen lamellaren liquidkristallinen Strukturen, sondern enthalten große Teile an ungequollenen Strukturen aus Perlglanzbildner und/oder Dispergiermittel, in welchen eine nicht ausreichende Menge Wasser gebunden ist. In **Fig. 1** ist eine solche kommerziell erhältliche Perlglanzdispersion abgebildet. Wie leicht zu erkennen ist, enthält das Konzentrat neben vollkommen durchgequollenen Bereichen, welche eine einheitliche körnige Struktur aufweisen, auch große inhomogene Bereiche, welche sich optisch deutlich abheben und welche nicht vollständig durchgequollen sind.

Dies ist durch die im Stand der Technik üblicherweise eingesetzten Batch-Verfahren bedingt, während derer das Vorhandensein von Toträumen sowie die Entstehung von Temperaturgradienten während der Abkühlung kaum vermieden werden können.

Dies führt dazu, dass Perlglanzdispersionen, die nach den im Stand der Technik bekannten Verfahren hergestellt werden, eine schlechte Lagerstabilität, insbesondere bei erhöhten Temperaturen ab ca. 35 °C, aufweisen. Werden die aus dem Stand der Technik bekannten Perlglanzdispersionen auf Temperaturen über diesem Punkt erwärmt, dann beginnt sich das vorhandene Bulkwasser in die ungequollenen Bereiche der Perlglanzdispersion einzulagern, es bilden sich neue lamellare Strukturen und die Viskosität der Zusammensetzung steigt bis um mehr als das 10fache. Die Zusammensetzungen können dann nicht mehr gut verarbeitet werden.

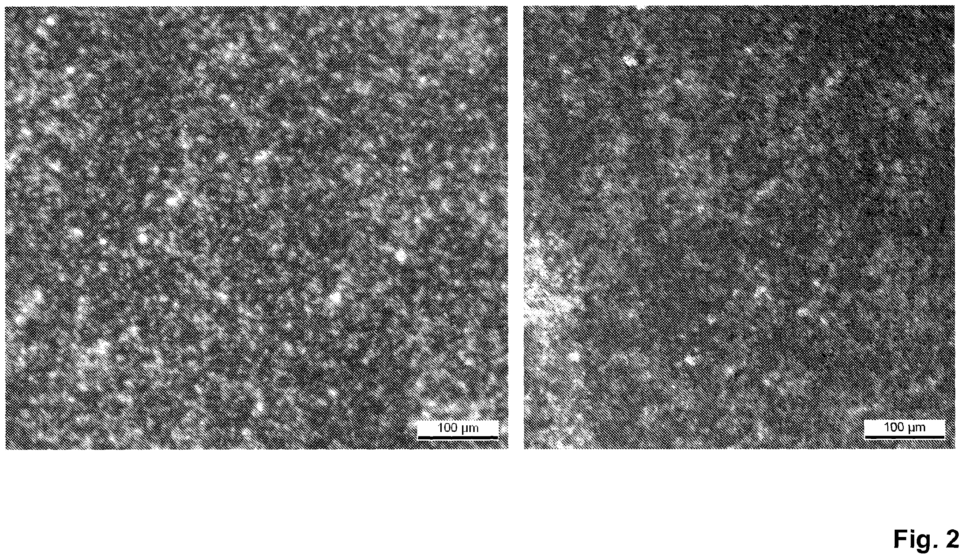

Die erfindungsgemäßen Perlglanzzusammensetzungen hingegen sind vollkommen durchgequollen. In **Fig. 2** ist ein erfindungsgemäßes Perlglanzkonzentrat gezeigt, welches sich durch eine hohe Homogenität und das Fehlen von ungequollenen Bereichen auszeichnet. Eine weitere Einlagerung von Bulkwasser und eine damit einhergehende Viskositätszunahme ist also nicht möglich. Auch durch eine Temperaturerhöhung auf 35 °C, bevorzugt über 45 °C, nimmt die Viskosität der erfindungsgemäßen Perlglanzkonzentrate um nicht mehr als 20 %, bevorzugt nicht mehr als 10 %, besonders bevorzugt nicht mehr als 5 %, zu. Die erfindungsgemäßen Perlglanzkonzentrate sind damit vorteilhaft lagerstabil und gut verarbeitbar.

Zur Steuerung der Viskosität einer Perlglanzdispersion werden im Stand der Technik oftmals Polyole zugesetzt. Die erfindungsgemäß hergestellten Perlglanzkonzentrate können hingegen vorteilhaft auf den Einsatz von Polyolen verzichten. In einer Ausgestaltungsform sind die erfindungsgemäßen Perlglanzkonzentrate daher frei von Polyolen. Im Sinne der Erfindung ist mit "frei von Polyolen" ein Polyolgehalt von weniger als 0,5 %, vorzugsweise weniger als 0,1 %, bevorzugt weniger als 0,05 % Polyole, bezogen auf das Gesamtvolumen des Perlglanzkonzentrats, gemeint. Ganz bevorzugt sind in den Zusammensetzungen keine freien Polyole nachweisbar.

Polyole im Sinne der vorliegenden Erfindung verfügen bevorzugt über zwei bis zwölf Kohlenstoffatome und zwei oder mehr Hydroxylgruppen. Daneben können die Polyole auch weitere funktionelle Gruppen umfassen. Beispiele für Polyole sind Glycerin, Ethylenglycol, Diethylenglycol, Propylenglycol, Butylenglycol, Pentylenglycol, Hexylenglycol, Polyethylenglycol sowie Zuckeralkohole wie z. B. Sorbitol oder Mannitol.

Mit den herkömmlichen diskontinuierlichen Verfahren sind die erfindungsgemäßen Perlglanzkonzentrate nur mit hohem Aufwand zu erhalten. Bestandteil der Erfindung ist daher ein Verfahren zur Herstellung der erfindungsgemäßen Perlglanzkonzentrate, welches die folgenden Schritte umfasst:
a) Einbringen eines Dispergiermittels ausgewählt aus Alkylethersulfaten und einer perlglanzbildenden Komponente ausgewählt aus einem oder mehreren Fettsäureglycolestern gemäß der allgemeinen Formel (I), 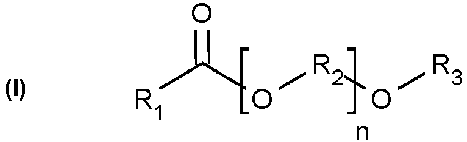
   wobei R₁ ein Alkylrest mit 12 bis 22 C-Atomen
   R₂ ein zweiwertiger Rest der Formel -C₂H₄- oder -C₃H₅-,
   R₃ = H oder ein Rest der Formel ist 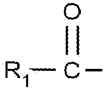 und n eine Zahl von 1 bis 10 ist,
   sowie ggf. Wasser in eine erste Rührstufe und Durchmischen bei 55 °C bis 90 °C in einem lamellaren Strömungsfluss, wobei die eingesetzte Wassermenge 35 Gew.-%, bevorzugt 30 Gew.-%, bezogen auf die Konzentration der eingesetzten Dispergiermittel und Perlglanzbildner nicht überschreitet, so dass sich eine lamellare lyotrope liquidkristalline Phase ausbildet,
b) Abkühlen der entstandenen Mischung über eine Kühlstufe auf eine Temperatur, bei der die perlglanzbildende Komponente in Form einer liquidkristallinen Lamellarstruktur auskristallisiert,
c) Intensive Vermischung der abgekühlten Mischung in einer zweiten Rührstufe und ggf. Verdünnung mit der Restmenge Wasser, wobei die Temperatur durch Verringerung der Rührleistung und/oder Kühlung der Rührstufe unter 45°C gehalten wird,
wobei Zusätze entweder in die erste oder die zweite Rührstufe eingebracht werden können.

Die erfindungsgemäß eingebrachten Dispergiermittel bzw. Perlglanzbildner können ein oder mehrere unterschiedliche Verbindungen aus den jeweiligen Gruppen enthalten.

Die Herstellung von Perlglanzsystemen verlangt eine genaue Kontrolle der Prozessparameter (Temperatur, Vermischungszeit, -dauer und -energie, Strömungsverlauf), da sonst die erhaltenen Perlglanzsysteme in ihren rheologischen, aber auch in ihren perlglänzenden Eigenschaften stark variieren. Die kontinuierliche Herstellung der Perlglanzsysteme erfolgt z. B. mithilfe der im Patent WO 2004/082817 beschriebenen Vorrichtung zur kontinuierlichen Herstellung von Emulsionen und Dispersionen durch eine gezielte Kombination von Rühr- und Kühlstufen.

Im Schritt a) werden die perlglanzbildenden Komponenten und das Dispergiermittel über geeignete Pumpen in die erste Rührstufe eingebracht und dort bei 55 °C bis 90 °C, bevorzugt 70 °C bis 80 °C vermischt. Sofern die Komponenten fließfähig sind, ist ein vorheriges Erhitzen nicht erforderlich.

Sind eine oder mehrere Komponenten nicht fließfähig, werden zur Erreichung einer homogenen Schmelze die Komponente(n) auf eine Temperatur von 5 - 20°C oberhalb ihres Schmelzpunktes erhitzt. Die Vermischung der Komponenten in der ersten Rührstufe erfolgt in einem lamellaren Strömungsfluss bei 55 °C bis 90 °C, bevorzugt 70 °C bis 80 °C. Ggf. erfolgt bereits in der ersten Rührstufe die Zugabe eines Teils oder der gesamten Menge des in der Rezeptur vorgesehenen Wassers. Liegen eine oder mehrere der in Schritt a) eingesetzten Komponenten als wässrige Lösung oder Suspension vor, kann die Zugabe von Wasser jedoch auch ganz entfallen.

Im ersten Verfahrensschritt a) werden die perlglanzbildenden Komponenten und das Dispergiermittel in eine kohärente lamellare liquidkristalline Phase überführt. Diese lamellare Phase besteht aus Doppelschichten der oberflächenaktiven Stoffe (Perlglanzbildner und Dispersionsmittel), worin die Moleküle dicht gepackt und parallel angeordnet sind, wobei die hydrophoben und hydrophilen Anteile der amphiphilen Verbindungen jeweils gleich ausgerichtet sind. Zwischen den hydrophilen Anteilen können Wassermoleküle eingeschoben werden, ein Prozess, der als Quellung bezeichnet wird. Die Menge Wasser, die durch Quellung aufgenommen kann, variiert je nach verwendeten Dispergiermittel und Perlglanzmittel.

Nach der Vermischung wird die vorgemischte Masse im Schritt b) über eine Kühlstufe so abgekühlt, dass die perlglanzgebende Komponente in Form einer flüssigkristallinen Lamellarstruktur auskristallisiert und sich der Perlglanzeffekt einstellt. Die Temperatur, auf welche die Mischung abgekühlt wird, beträgt dabei vorzugsweise unter 45 °C, bevorzugt 20 °C bis 42 °C, besonders bevorzugt 30 °C bis 40 °C.

Abschließend wird im Schritt c) in einer weiteren Rührstufe die gekühlte Masse unter mäßigem Rühren intensiv vermischt und ggf. mit der Restmenge Wasser verdünnt. Dadurch wird die Viskosität des Konzentrats vorteilhaft soweit gesenkt, dass sich das Konzentrat gut weiterverarbeiten lässt. Dabei wird die Temperaturerhöhung bei der Vermischung bevorzugt so kontrolliert, dass die Temperatur der Produktes < 45°C bleibt. Dies wird vorzugsweise entweder durch Verringerung der Rührleistung und/oder durch intensives Kühlen der Rührstufe erreicht.

Durch die kontinuierliche Verarbeitung der Komponenten des Perlglanzkonzentrats wird der Einsatz von klein dimensionierten Mischkammern ermöglicht. Vorteilhaft wird dadurch der Energieeintrag durch das Rührwerk minimiert und die Geschwindigkeit der Durchmischung und Emulsionsbildung maximiert.

Ebenso ist auch die Kühlstufe vorteilhaft niedrig dimensioniert, wodurch die Bildung von Temperaturgradienten minimiert wird, ohne dass die zu kühlende Mischung gerührt werden muss. Dadurch kommt es zu einer raschen und zufriedenstellenden Bildung einer lamellaren liquidkristallinen Phase in dem herzustellenden Perlglanzkonzentrat. Bevorzugt wird die Mischung daher in der Kühlstufe des erfindungsgemäßen Verfahrens daher nicht gerührt.

Durch das Nachrühren in der zweiten Rührstufe und/oder den Zusatz von Wasser wird im Schritt c) die bis dahin kohärente lamellare liquidkristalline Phase in kleine plättchenförmige Partikel "zerrissen", die in dem Anteil Wasser, der nicht interlamellar gebunden ist, dispergiert sind. Dabei weisen die Partikel der erfindungsgemäßen Perlglanzkonzentrate vorteilhaft eine sehr homogene Teilchengrößenverteilung in Form einer Gauß-Verteilung auf. Die sich daraus ergebende Partikelgröße kann durch Wahl der Rührgeschwindigkeit und Verweildauer zwischen 0,2 und 20 µm gezielt eingestellt werden.

Vorzugsweise wird das erfindungsgemäße Verfahren bei Normaldruck oder bei Drücken bis 10 bar, bevorzugt bis 5 bar, durchgeführt.

Sollen dem Perlglanzkonzentrat zweckmäßige Zusätze zugefügt werden, so können diese in die erste oder in die zweite Rührstufe eingebracht werden. Solche zweckmäßigen Zusätze können beispielsweise Elektrolyte, z. B. Natriumchlorid, Kaliumchlorid und/oder Magnesiumchlorid, Konservierungsmittel, z. B. Ameisensäure, oder auch Puffersubstanzen und dergleichen sein.

Die Erfindung wird durch die folgenden Figuren und Ausführungsbeispiele näher erläutert, ohne auf diese beschränkt zu sein. Dabei zeigt
Fig. 1 eine lichtmikroskopische Aufnahme eines herkömmlichen Perlglanzdispersionen mit ungequollenen Bereichen (gekennzeichnet durch gestrichelte Linie) (Mess-Bedingung: linear polarisiertes Licht, 100fache Vergrößerung, Polfilter) und
Fig. 2 lichtmikroskopische Aufnahmen erfindungsgemäßer Perlglanzkonzentrate, die vollständig durchgequollen sind und keine ungequollene Bereiche aufweisen (Mess-Bedingungen: linear polarisiertes Licht, 100fache Vergrößerung, Polfilter).

### Ausführungsbeispiel 1: Beispielhafte Rezepturen für die erfindungsgemäß hergestellten Perlglanzkonzentrate

| **Komponente** | **Handelsbezeichnung** | **Gew.-%** | | | | | |
|---|---|---|---|---|---|---|---|
| | | **1** | **2** | **3** | **4** | **5** | **6** |
| **Natriumlaurylethersulfat*** | Galaxy LES 70 | 70 | 80 | 80 | 80 | 85 | 80 |
| **Ethylenglycoldistearat** | Galaxy 610 | 30 | 15 | 10 | 2,5 | | |
| **Kokosfettsäure-monoethanolamid** | Galaxy 100 | | 5 | 10 | 17,5 | 15 | 20 |

| | | | | | | | |
|---|---|---|---|---|---|---|---|
| * = 70 %ige wässrige Lösung | | | | | | | |

### Ausführungsbeispiel 2: Herstellung eines Perlglanzkonzentrats

Das Verfahren wird in einer zweistufigen Vorrichtung durchgeführt, wobei Phase A und Phase B getrennt in die erste Rührstufe eingebracht werden, und der Austrag über eine Kühlstufe abgekühlt und in die zweite Rührstufe geführt wird. Die angegebenen Prozentangaben sind Gewichtsprozent.

Durch die Variation der Verweilzeiten in den einzelnen Stufen kann die pro Zeiteinheit produzierte Menge gezielt gesteuert werden. Bei Verweilzeiten in Stufe 1 - Kühlstufe - Stufe 2 von 20 s - 53 s - 20 s können etwa 60 kg Perlglanzkonzentrat pro Stunde, bei 16 s - 41 s - 16 s etwa 80 kg Perlglanzkonzentrat pro Stunde und bei 10 s - 26 s - 10 s etwa 120 kg Perlglanzkonzentrat pro Stunde produziert werden.

| **Komponente** | **Handelsbezeichnung** | |
|---|---|---|
| **Phase A:** | | |
| Natriumlaurylethersulfat* | Galaxy LES 70 | 80 % |
| | | |

| **Phase B:** | | |
|---|---|---|
| Ethylenglycoldistearat | Galaxy 610 | 10 % |
| Kokosfettsäure-monoethanolamid | Galaxy 100 | 10 % |
| | | |
| **Umfangsgeschwindigkeit Stufe 1 [m/s]** | | 0,1 - 15 |
| **Umfangsgeschwindigkeit Stufe 2 [m/s]** | | 0,1 - 15 |
| **Verweilzeit Stufe 1 [s]** | | 10 - 20 |
| **Verweilzeit Kühlstufe [s]** | | 30 - 60 |
| **Verweilzeit Stufe 2 [s]** | | 10 - 20 |

### Ausführungsbeispiel 3: Versuche zur Lagerstabilität

Es wurde das Viskositätsverhalten von Perlglanzdispersionen unter Schüttelbedingungen auf einem Orbitalschüttler (45°C bei 150 RPM) untersucht. Die Ergebnisse für zwei erfindungsgemäße Zusammensetzungen (1, 2; entsprechen den Zusammensetzungen 1 und 2 aus Ausführungsbeispiel 1) sowie fünf kommerziell erhältliche Vergleichszusammensetzungen (3 bis 7) sind in der folgenden Tabelle wiedergegeben. Die Viskositätszunahme ist in Prozent der Anfangsviskosität der jeweiligen Zusammensetzung wiedergegeben. Die Viskositätsachse ist aus Darstellungsgründen logarithmisch eingeteilt.

| **Zusammensetzung** | **6h** | **24h** |
|---|---|---|
| **1** | 0 | 0 |
| **2** | 0 | 0 |
| **3** | 50 | 135 |
| **4** | 1300 | 1330 |
| **5** | 129 | 344 |
| **6** | 88 | 182 |
| **7** | 0 | 33 |

Es ist deutlich zu erkennen, dass die herkömmlichen Zusammensetzungen unter Versuchsbedingungen eine starke Viskositätszunahme aufweisen, die Zusammensetzungen 1 und 2 hingegen nicht. Die Viskosität der Zusammensetzungen wurde mittels eines Kegel-Platte-Rheometers bestimmt.

## Patentansprüche

1. Verfahren zur Herstellung eines pumpbaren wässrigen Perlglanzkonzentrats, umfassend die Schritte
a) Einbringen eines Dispergiermittels ausgewählt aus Alkylethersulfaten und
einer perlglanzbildenden Komponente ausgewählt aus einem oder mehreren Fettsäureglycoiestern gemäß der allgemeinen Formel (I), 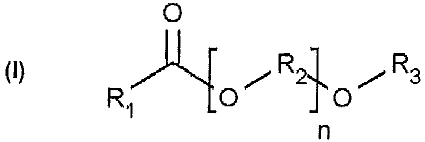
wobei R₁ ein Alkylrest mit 12 bis 22 C-Atomen
R₂ ein zweiwertiger Rest der Formel -C₂H₄- oder -C₃H₅-,
R₃ = H oder ein Rest der Formel ist 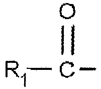
und n eine Zahl von 1 bis 10 ist,
sowie ggf. Wasser in eine erste Rührstufe und Durchmischen bei 55 °C bis 90 °C in einem lamellaren Strömungsfluss, wobei die eingesetzte Wassermenge 35 Gew.-% bezogen auf die Konzentration der eingesetzten Dispergiermittel und Perlglanzbildner nicht überschreitet,
b) Abkühlen der Mischung über eine Kühlstufe auf unter 45°C, wobei die perlglanzbildende Komponente in Form einer liquid kristallinen Lamellarstruktur auskristallisiert,
c) Intensive Vermischung der abgekühlten Mischung in einer zweiten Rührstufe und ggf. Verdünnung mit der Restmenge Wasser, wobei die Temperatur durch Verringerung der Rührleistung und/oder Kühlung der Rührstufe unter 45°C gehalten wird,
wobei Zusätze entweder in die erste oder die zweite Rührstufe eingebracht werden können.

2. Verfahren nach Anspruch 1, wobei im Schritt a) die Komponenten über geeignete Pumpen in die erste Rührstufe eingebracht werden und dort bei 70 °C bis 80 °C vermischt werden.

3. Verfahren nach einem oder mehreren der Ansprüche 1 bis 2, wobei die Temperatur, auf welche die Mischung abgekühlt wird, dabei 20 °C bis 42 °C, bevorzugt 30 °C bis 40 °C beträgt.

4. Verfahren nach einem oder mehreren der Ansprüche 1 bis 3, wobei das Verfahren bei Drucken bis 10 bar, bevorzugt bis 5 bar, durchgeführt wird.

5. Verfahren nach einem oder mehreren der Ansprüche 1 bis 4, wobei die Zusätze ausgewählt aus Elektrolyten, Konservierungsmitteln und Puffersubstanzen sind.

6. Verfahren nach einem oder mehreren der Ansprüche 1 bis 5, wobei als Alkylethersulfate die Sulfate von Fettalkoholen mit einer Kettenlänge von 8 bis 16 C-Atomen eingesetzt werden.

7. Verfahren nach Anspruch 6, wobei die Alkylethersulfate aus Natriumlaurylethersulfat, Ammoniumlaurylethersulfat, Triethylaminlaurylethersulfat, Triethanolaminlaurylethersulfat, Magnesiumlaurylethersulfat, Natrium C12-13 Pareth Sulfat (Sodium C12-13 Pareth Sulfate), und das Na-Salz von Laurylmyristylethersulfat ausgewählt sind.

8. Verfahren nach einem oder mehreren der Ansprüche 1 bis 7, wobei R₁ ein gesättigter oder ungesättigter Alkylrest mit 12 bis 18 C-Atomen ist, R₂ -C₂H₄ ist, R₃ R₁-CO- ist, und n eine Zahl von 1 bis 3 ist.

9. Verfahren nach einem oder mehreren der Ansprüche 1 bis 8, wobei falls der Alkylrest R₁ ungesättigt ist, enthält er 1 bis 3 Doppelbindungen.

10. Verfahren nach einem oder mehreren der Ansprüche 1 bis 7, wobei die perlglanzbildende Komponenten Mono- und Diester des Ethylenglycols, Diethylenglycols oder Triethylenglycols mit Palmitinsäure, Stearinsäure, Ölsäure, Talgfettsäure, Cocosfettsäure und/oder anderen Fettsäuren sind.

11. Verfahren nach einem oder mehreren der Ansprüche 1 bis 7, wobei die perlglanzbildende Komponenten aus Ethylenglycolmonostearat, Ethylenglycoldistearat, Diethylenglycoldistearat ausgewählt sind.

12. Verfahren nach einem oder mehreren der Ansprüche 1 bis 11, wobei das pumpbare wässrige Perglanzkonzentrat frei von Polyolen ist.

## Claims

1. Process for the production of a pumpable aqueous pearlescent concentrate, comprising the steps of
a) introduction of a dispersant selected from alkyl ether sulphates and
of a pearlescent component selected from one or more fatty acid glycol esters according to the general formula (I), 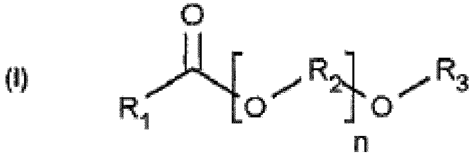
in which R₁ is an alkyl radical with 12 to 22 C atoms,
R₂ is a divalent radical of the formula -C₂H₄- or -C₃H₅-,
R₃ = H or is a radical of the formula 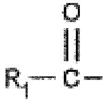
and n is a number from 1 to 10,
and optionally water in a first stirring step and thorough mixing at 55°C to 90°C in a lamellar fluid flow, in which the amount of water employed does not exceed 35% by weight based on the concentration of the dispersants and pearlescers employed,
b) cooling of the mixture by means of a cooling step to below 45°C, in which the pearlescent component crystallizes in the form of a liquid crystalline lamellar structure,
c) intensive mixing of the cooled mixture in a second stirring step and optionally dilution with the remaining amount of water, in which the temperature is kept below 45°C by reduction of the stirring power and/or cooling of the stirring step,
in which additives can be introduced either in the first or the second stirring step.

2. Process according to Claim 1, in which in step a) the components are introduced into the first stirring step via suitable pumps and are mixed in that step at 70°C to 80°C.

3. Process according to one or more of Claims 1 to 2, in which the temperature to which the mixture is cooled is 20°C to 42°C, preferably 30°C to 40°C.

4. Process according to one or more of Claims 1 to 3, in which the process is carried out at pressures of up to 10 bar, preferably up to 5 bar.

5. Process according to one or more of Claims 1 to 4, in which the additives are selected from electrolytes, preservatives and buffer substances.

6. Process according to one or more of Claims 1 to 5, in which the alkyl ether sulphates employed are the sulphates of fatty alcohols having a chain length of 8 to 16 C atoms.

7. Process according to Claim 6, in which the alkyl ether sulphates are selected from sodium lauryl ether sulphate, ammonium lauryl ether sulphate, triethylamine lauryl ether sulphate, triethanolamine lauryl ether sulphate, magnesium lauryl ether sulphate, Sodium C12-13 Pareth Sulfate, and the Na salt of lauryl myristyl ether sulphate.

8. Process according to one or more of Claims 1 to 7, in which R₁ is a saturated or unsaturated alkyl radical with 12 to 18 C atoms, R₂ is -C₂H₄, R₃ is R₁-CO-, and n is a number from 1 to 3.

9. Process according to one or more of Claims 1 to 8, in which, if the alkyl radical R₁ is unsaturated, it contains 1 to 3 double bonds.

10. Process according to one or more of Claims 1 to 7, in which the pearlescent components are mono- and diesters of ethylene glycol, of diethylene glycol or of triethylene glycol with palmitic acid, stearic acid, oleic acid, tallow fatty acid, coconut fatty acid and/or other fatty acids.

11. Process according to one or more of Claims 1 to 7, in which the pearlescent components are selected from ethylene glycol monostearate, ethylene glycol distearate, diethylene glycol distearate.

12. Process according to one or more of Claims 1 to 11, in which the pumpable aqueous pearlescent concentrate is free of polyols.

## Revendications

1. Procédé de fabrication d'un concentré nacré aqueux pompable, comprenant les étapes suivantes :
a) l'introduction d'un agent de dispersion choisi parmi les alkyl-éther-sulfates et d'un composant nacrant choisi parmi un ou plusieurs esters de glycol d'acides gras selon la formule générale (I) 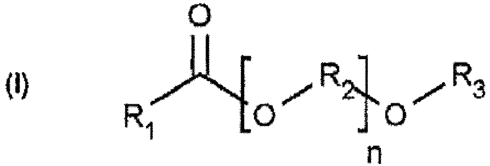
dans laquelle R₁ est un radical alkyle de 12 à 22 atomes C,
R₂ est un radical bivalent de formule -C₂H₄- ou -C₃H₅-,
R₃ = H ou un radical de formule 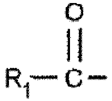 et n est un nombre de 1 à 10,
ainsi qu'éventuellement de l'eau dans une première étape d'agitation et le mélange à une température de 55 °C à 90 °C dans un flux d'écoulement laminaire, la quantité d'eau utilisée ne dépassant pas 35 % en poids, par rapport à la concentration de l'agent de dispersion et de l'agent nacrant utilisés,
b) le refroidissement du mélange par une étape de refroidissement à moins de 45 °C, le composant nacrant cristallisant sous la forme d'une structure lamellaire cristalline liquide,
c) le mélange intensif du mélange refroidi dans une deuxième étape d'agitation et éventuellement la dilution avec la quantité résiduelle d'eau, la température étant maintenue en dessous de 45 °C par réduction de la puissance d'agitation et/ou refroidissement de l'étape d'agitation,
des additifs pouvant être introduits dans la première ou dans la deuxième étape d'agitation.

2. Procédé selon la revendication 1, dans lequel, à l'étape a), les composants sont introduits dans la première étape d'agitation par des pompes appropriées, et y sont mélangés à une température de 70 °C à 80 °C.

3. Procédé selon une ou plusieurs des revendications 1 à 2, dans lequel la température à laquelle le mélange est refroidi est de 20 °C à 42 °C, de préférence de 30 °C à 40 °C.

4. Procédé selon une ou plusieurs des revendications 1 à 3, dans lequel le procédé est réalisé à des pressions de jusqu'à 10 bar, de préférence de jusqu'à 5 bar.

5. Procédé selon une ou plusieurs des revendications 1 à 4, dans lequel les additifs sont choisis parmi les électrolytes, les conservateurs et les substances tampons.

6. Procédé selon une ou plusieurs des revendications 1 à 5, dans lequel des sulfates d'alcools gras ayant une longueur de chaîne de 8 à 16 atomes C sont utilisés en tant qu'alkyl-éther-sulfates.

7. Procédé selon la revendication 6, dans lequel les alkyl-éther-sulfates sont choisis parmi le lauryl-éther-sulfate de sodium, le lauryl-éther-sulfate d'ammonium, le lauryl-éther-sulfate de triéthylamine, le lauryl-éther-sulfate de triéthanolamine, le lauryl-éther-sulfate de magnésium, le pareth-sulfate en C12-13 de sodium (Sodium C12-13 Pareth Sulfate) et le sel de Na de laurylmyristyl-éther-sulfate.

8. Procédé selon une ou plusieurs des revendications 1 à 7, dans lequel R₁ est un radical alkyle saturé ou insaturé de 12 à 18 atomes C, R₂ est -C₂H₄, R₃ est R₁-CO- et n est un nombre de 1 à 3.

9. Procédé selon une ou plusieurs des revendications 1 à 8, dans lequel, si le radical alkyle R₁ est insaturé, il contient 1 à 3 doubles liaisons.

10. Procédé selon une ou plusieurs des revendications 1 à 7, dans lequel les composants nacrants sont des mono- ou diesters d'éthylène glycol, de diéthylène glycol ou de triéthylène glycol avec de l'acide palmitique, de l'acide stéarique, de l'acide oléique, de l'acide gras de suif, de l'acide gras de coco et/ou d'autres acides gras.

11. Procédé selon une ou plusieurs des revendications 1 à 7, dans lequel les composants nacrants sont choisis parmi le monostéarate d'éthylène glycol, le distéarate d'éthylène glycol, le distéarate de diéthylène glycol.

12. Procédé selon une ou plusieurs des revendications 1 à 11, dans lequel le concentré nacré aqueux pompable est exempt de polyols.
